Europäisches Patentamt

European Patent Office

Office européen des brevets

(11) Veröffentlichungsnummer: **0 485 836 A2**

# EUROPÄISCHE PATENTANMELDUNG

(21) Anmeldenummer: **91118728.4**

(51) Int. Cl.5: **G01N 21/35**, G01N 33/44

(22) Anmeldetag: **04.11.91**

(30) Priorität: **14.11.90 DE 4036201**

(43) Veröffentlichungstag der Anmeldung:
**20.05.92 Patentblatt 92/21**

(84) Benannte Vertragsstaaten:
**BE CH DE FR GB IT LI**

(71) Anmelder: **BAYER AG**

**W-5090 Leverkusen 1 Bayerwerk(DE)**

(72) Erfinder: **Wolf, Udo, Dr.**
**Uerdinger Strasse 469**
**W-4150 Krefeld(DE)**
Erfinder: **Dohmen, Hans**
**Birmesstrasse 14**
**W-4150 Krefeld(DE)**
Erfinder: **Diefendahl, Jürgen**
**Hochkamer Strasse 98a**
**W-4133 Neukirchen-Vluyn(DE)**

(54) **Spektroskopisches Analyseverfahren für Kunststoffgemische.**

(57) Es wird ein Verfahren zur Analyse von Kunststoffgemischen mittels Infrarot-Transmissionsspektroskopie beschrieben, bei dem die Spektren bei gleichzeitiger Rotation der Proben aufgenommen werden. Durch diese Maßnahme wird die Genauigkeit und Reproduzierbarkeit der Spektren und damit auch der Bestimmung einzelner Komponenten des Kunststoffgemisches verbessert.

EP 0 485 836 A2

Die Erfindung betrifft ein Verfahren zur Analyse einer Probe aus Kunststoffgemischen durch Bestimmung von Infrarot-Transmissionsspektren.

Das Gebiet der Kunststofferzeugung und -verarbeitung ist gekennzeichnet durch ständig steigende Anforderungen an die Qualität einzelner Produkte, Zur Erfüllung dieser Qualitätsanforderungen bedarf es der präzisen Einhaltung vorgegebener Spezifikationen, insbesondere der Konzentrationen einzelner Bestandteile in Kunststoffgemischen. Kunststoffgemische im Sinne der Erfindung können unter anderem sein: Copolymere, Polymerblends oder füllstoff- oder faserverstärkte Kunststoffe. Ein weiteres Anwendungsfeld der Erfindung stellt die Bestimmung der Konzentrationen von Additiven dar. Solche Additive sind beispielsweise Entformungsmittel oder Weichmacher. Die Konzentrationen von Entformungsmitteln betragen typischerweise weniger als 1 %; trotzdem ist es häufig erforderlich, ihren Masseanteil am Stoffgemisch genau zu bestimmen.

Es sind bereits verschiedene spektroskopische Verfahren zur Bestimmung der Zusammensetzung von Kunststoffgemischen bekannt. Ein häufig angewendetes Verfahren besteht darin, zunächst eine Lösung des Kunststoffes herzustellen und anschließend ein Transmissionsspektrum der Lösung aufzunehmen. Anhand von Absorptionsbanden, die für die zu bestimmenden Komponenten charakteristisch sind, können nach zuvor erfolgter Eichung unter Anwendung des Lambert-Beerschen Gesetzes oder neuerer numerischer Auswerteverfahren die Konzentrationen der zu bestimmenden Komponenten ermittelt werden. Dieses Verfahren besitzt jedoch den Nachteil, daß für einige moderne Hochleistungskunststoffe, z.B. für Polyphenylensulfid, unterhalb von 150°C keine Lösungsmittel bekannt sind.

Weiter ist aus US-PS 4 717 827 ein Verfahren bekannt, bei dem die Transmissionsmessung des zu analysierenden Kunststoffes an einer geschmolzenen Probe vorgenommen wird Dieses Verfahren ist jedoch vor allem zur on-line Produktionskontrolle eines Produktes geeignet, weniger für die Messung einer Vielzahl unterschiedlicher Proben in kurzer Zeit, da die Säuberung und erneute Füllung der Meßzelle relativ aufwendig ist, Für die Analyse einer Vielzahl unterschiedlicher Proben ist es am vorteilhaftesten, die Transmissionsspektren von Preßfilmen der zu analysierenden Kunststoffgemische zu bestimmen. Preßfilme werden auf bekannte Weise durch Verpressen eines Kunststoffgemisches bei einer Temperatur des Preßwerkzeuges oberhalb der Schmelztemperatur des zu analysierenden Gemisches hergestellt. Ihre Dicke beträgt üblicherweise zwischen 1 und 500 μm. Ferner können für spektroskopische Messungen auch durch Spritzgußverarbeitung hergestellte Körper mit typischen Wandstärken zwischen 0,1 und 100 mm verwendet werden. Da sowohl solchermaßen hergestellte Preßfilme oder Spritzgußkörper als auch der Analysenstrahl handelsüblicher Spektrometer häufig Anisotropien oder Inhomogenitäten aufweisen, unterliegen die Spektren dieser Kunststofffilme erheblichen Schwankungen, die sich entsprechend negativ auf die Genauigkeit der Konzentrationsbestimmung einzelner Komponenten im Kunstoffgemisch auswirken.

Es bestand daher die Aufgabe, ein Verfahren zur Analyse von Kunstoffgemischen durch Transmissionsspektroskopie an festen Proben mit erhöhter Genauigkeit zu finden.

Das neue Verfahren löst die Aufgabe dadurch, daß die Spektren bei gleichzeitiger Rotation der Proben und Massenanteile einzelner Bestandteile des Kunststoffgemisches nach an sich bekannten Verfahren aus den Spektren bestimmt werden.

Die Rotation der Probe bei der Aufnahme von Infrarot-Transmissionsspektren ist als solche bekannt. Sie wird bisher hauptsächlich bei der Bestimmung von molekularen Vorzugsrichtungen in Polymerproben verwendet. Bei der Entwicklung des neuen Verfahrens wurde festgestellt, daß die Rotation der Probe bei gleichzeitiger Aufnahme der Transmissionsspektren die Qualität der Konzentrationsbestimmung deutlich verbessert. Dies gilt unabhängig von dem Zeitpunkt, an dem aus der Vielzahl der Messungen ein Mittelwert bestimmt wird. Üblicherweise wird zunächst aus der Vielzahl der aufgenommenen Spektren ein mittleres Spektrum bestimmt, anhand dessen nach den oben bereits beschriebenen Verfahren die Konzentration der zu bestimmenden Komponente ermittelt werden. Es lassen sich jedoch ebenfalls zunächst die Konzentration aus den einzelnen Spektren bestimmen. Die so erhaltenen Werte werden dann zur Berechnung des Mittelwertes verwendet. Beide Mittlungsverfahren sind insbesondere im Bereich der Fourier-Transform-Infrarotspektroskopie gut bekannt.

Eine besonders bevorzugte Variante des neuen Verfahrens ist dadurch gekennzeichnet, daß die Winkelgeschwindigkeit der Rotation so eingestellt wird, daß die Periodendauer der Rotation größer als die Meßzeit zur Bestimmung eines der Transmissionsspektren ist.

Die Winkelgeschwindigkeit der Rotation der Probe um die optische Achse des Spektrometers ist eine wesentliche Größe bei der Durchführung des Verfahrens. Sie dient bei vorgegebenen Parametern (zur Verfügung stehender Meßzeit, gewünschte Meßgenauigkeit) der Optimierung des Verfahrens. Eine zu langsam gewählte Winkelgeschwindigkeit verlängert die Zeit, die zum Durchführen des neuen Verfahrens notwendig ist. Wählt man die Winkelgeschwindigkeit zu groß, insbesondere über 1.000 Hz, so bedarf es eines besonderen technischen Aufwandes, um das Fourier-Spektrometer vor den Einflüssen der mechanischen Bewegung abzuschirmen. Bewährt haben sich Winkelgeschwindigkeiten zwischen 1 und 3 Hz.

Eine weitere bevorzugte Variante des Verfahrens ist dadurch gekennzeichnet, daß die Winkelgeschwindigkeit der Rotation während der Bestimmung der Spektren kontinuierlich und/oder diskontinuierlich verändert wird,

Dieses Verfahren ist besonders dann vorteilhaft, wenn man die einzelnen Transmissionsspektren der Polymerprobe unter verschiedenen, definierten Winkeln $\alpha_i$ bestimmen will, Durch Synchronisation des Spektrometers mit der Probendrehvorrichtung läßt sich beispielsweise die Probe vor Aufnahme eines Spektrums um jeweils $\delta\alpha = 10°$ drehen und zur Aufnahme des Spektrums wieder abstoppen. Mit den solchermaßen gewonnenen 36 Werten bei Vollendung einer vollen Umdrehung lassen sich die oben angeführten Mittlungsverfahren durchführen, Dieses Verfahren dient insbesondere der Minimierung der Gesamtmeßzeit für eine einzelne Kunststoffprobe.

Eine weitere bevorzugte Variante des Verfahrens ist dadurch gekennzeichnet, daß die Transmissionsspektren in dem Wellenzahlbereich zwischen 400 cm$^{-1}$ und 4000 cm$^{-1}$ aufgenommen werden, In diesem Wellenzahlbereich liegen die charakteristischen Banden der meisten Moleküle,

Im folgenden wird das neue Verfahren anhand von Beispielen und einer Zeichnung näher erläutert.

Es zeigen:

Fig. 1 Ausschnitt aus dem Absorptionsspektrum von Polycarbonat (extrudierter Film),

Fig. 2 Vergleich zwischen dem vorgegebenen Kautschukgehalt und dem nach dem neuen Verfahren ermittelten Kautschukgehalt mehrerer Proben eines Kunststoff-Compounds auf Polyamid-Basis,

Fig. 3 Vergleich zwischen vorgegebener und spektroskopisch ermittelter Konzentration eines typischen Entformungswachses in Polybutylenterephthalat.

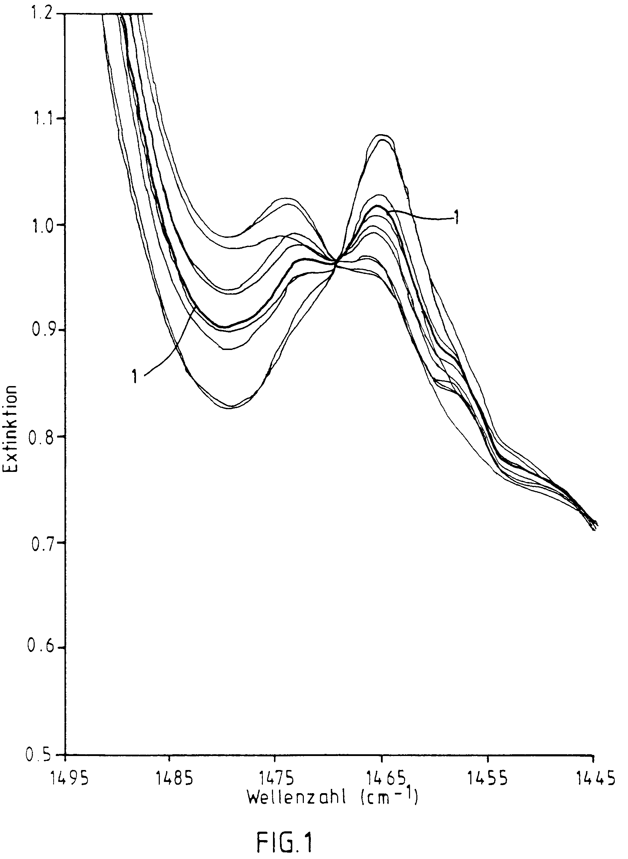

Die in Fig. 1 gezeigten neun Infrarot-Transmissionsspektren von Polycarbonatfilmen aus einer einzigen Grundmenge wurden mit Hilfe eines handelsüblichen Fourier-Transform-Spektrometers aufgenommen. Das Absorptionsmaximum bei 1.463 cm$^{-1}$ zeigt beispielsweise Abweichungen von über 10 %. zwischen den Absorptionswerten der einzelnen Proben bei dieser Wellenzahl. Diese Schwankungen übertragen sich selbstverständlich auf jedes Verfahren zur Bestimmung der Konzentration von einzelnen Komponenten, denen eine solche Messung des Infrarot-Spektrums zugrunde liegt. Bei Anwendung des neuen Verfahrens, d.h. bei Rotation der einzelnen Proben während der Aufnahme, sind die Spektren der einzelnen Proben im Rahmen der Meßgenauigkeit des Spektrometers ununterscheidbar (Spektrum 1).

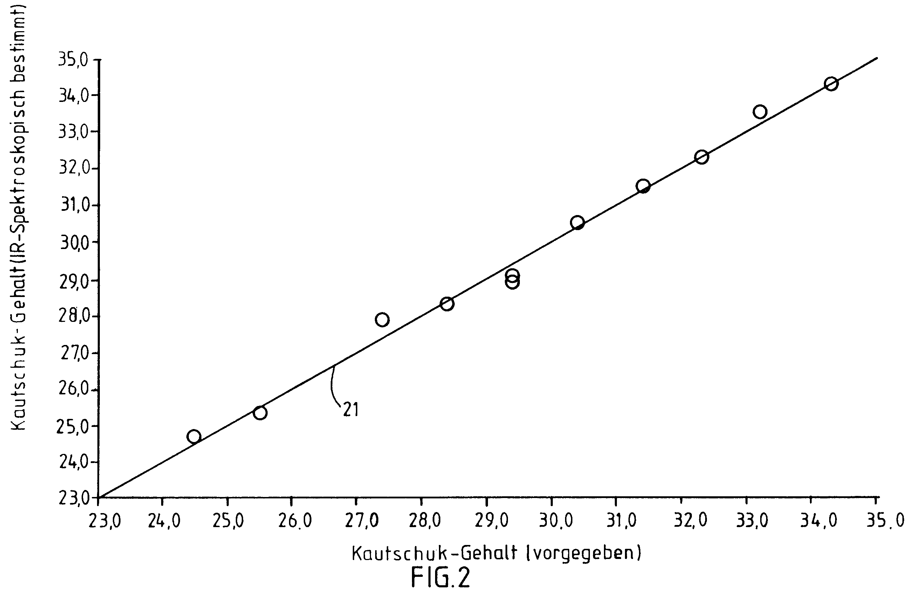

Weiter wurde das neue Verfahren beispielsweise bei der Bestimmung des Kautschukanteils in schlagzähmodifiziertem Polyamid-6 eingesetzt. Bei dieser Konzentrationsbestimmung ging es um das Problem, einen relativ hohen Anteil einer Substanz im Kunststoffgemisch mit hoher Genauigkeit zu bestimmen, So beträgt die Konzentration von Kautschuk in diesen schlagzähmodifizierten Kunststoffen typischerweise zwischen 20 und 40 %. Zur Messung wurden 12 Proben mit bekanntem Kautschukgehalt mit Hilfe des neuen Verfahrens untersucht, Dabei wurden heißverpreßte Filme des Kunststoffgemisches mit einer Dicke zwischen 20 und 50 $\mu$m in eine drehbare Probenhalterung gespannt und mit einer Rotationsfrequenz von 1,5 Hz gedreht. Während dieser Drehung wurden etwa 100 Interferogramme mit Hilfe eines üblichen Fourier-Transform-Infrarotspektrometers (Nicolet 60 SX) akkumuliert. Das nach Ausführung der Fourier-Transformation gewonnene Spektrum wurde nach üblichen Verfahren ausgewertet, Dazu wurde die Absorptionsbande bei 1.365 cm$^{-1}$ als Referenzbande zur Berücksichtigung der Schichtdicke verwendet und die Butadien-Absorption bei 911 cm$^{-1}$ zur Bestimmung der Kautschukkonzentration herangezogen. Fig. 2 zeigt die Meßergebnisse in Relation zur Identitätsgerade 21. Die Standardabweichung des Verfahrens beträgt etwa 0,05 %.

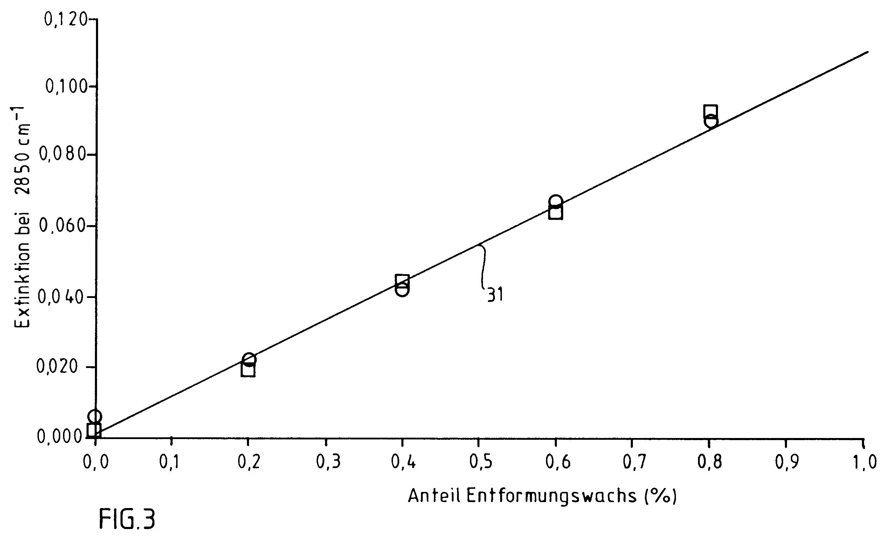

In einem weiteren Beispiel wurde die Konzentration eines Entformungsmittels in Polybutylenterephthalat (PBT) bestimmt, Da die Konzentration solcher Additive, bezogen auf die Gesamtmenge des Kunststoffes, relativ klein ist (IR: 0,5 %), ging es in diesem Beispiel um die genaue Bestimmung der Konzentration eines Stoffes mit geringem Anteil an der Gesamtzusammensetzung. Das neue Verfahren wurde an Proben mit definiertem Gehalt an Entformungswachs durchgeführt, die unter Verwendung eines Laborkneters hergestellt wurden. Zur Messung der IR-Transmissionsspektren wurden Preßfilme von einer Dicke von 50 $\mu$m (Meßpunkte durch Quadrate gekennzeichnet) und 100 $\mu$m (Meßpunkte durch Kreise gekennzeichnet) hergestellt. Diese wurden in eine Probenhalterung eingespannt, deren Rotationsfrequenz während der Messung 2 Hz betrug. Als Absorptionsbande des Entformungsmittels wurde die C-H-Streckschwingung bei 2.850 cm$^{-1}$ verwendet. Fig. 3 zeigt die Eichgerade 31, die aufgrund dieser Messung erhalten wurde, Ihre Genauigkeit beträgt ± 0,02 %.

Um das Verfahren zu verbessern, wurde von den aufgenommenen Spektren der Probe mit Additiv das im Spektrometer gespeicherte Spektrum der reinen Grundsubstanz (hier; PBT) in Abhängigkeit von der Probendicke substrahiert und das so entstandene Differenzspektrum bei der Bestimmung der Konzentration zugrundegelegt.

**Patentansprüche**

1. Verfahren zur Analyse einer Probe eines Kunststoffgemisches durch Bestimmung von Infrarot-Transmissionsspektren, dadurch gekennzeichnet, daß die Spektren bei gleichzeitiger Rotation der Probe bestimmt werden und Massenanteile einzelner Bestandteile des Kunststoffgemisches nach an sich bekannten Verfahren aus den so erhaltenen Spektren bestimmt werden,

2. Verfahren nach Anspruch 1, dadurch gekennzeichnet, daß die Winkelgeschwindigkeit der Rotation der Probe so eingestellt wird, daß die Periodendauer der Rotation größer als die Meßzeit zur Bestimmung eines Transmissionsspektrums ist.

3. Verfahren nach den Ansprüchen 1 bis 2, dadurch gekennzeichnet, daß die Winkelgeschwindigkeit der Rotation während der Bestimmung der Spektren kontinuierlich und/oder diskontinuierlich verändert wird.

4. Verfahren nach den Ansprüchen 1-3, dadurch gekennzeichnet, daß die Transmissionsspektren in dem Wellenzahlbereich zwischen 400 cm$^{-1}$ und 4000 cm$^{-1}$ aufgenommen werden.

FIG.1

FIG.2

EP 0 485 836 A2

FIG.3

EP 0 485 836 A2